# EUROPEAN PATENT APPLICATION

(11) **EP 3 228 701 A1**
(43) Date of publication of application: **11.10.2017**
(21) Application number: 16382147.3
(22) Date of filing: 05.04.2016
(51) Int. Cl.: C12N 5/0783, A61K 35/17, A61K 49/00, A61K 39/00

(54) **METHOD FOR PREPARING LYMPHOCYTES CAPABLE OF INFILTRATING A SOLID TUMOR, LYMPHOCYTES OBTAINABLE BY SAID METHOD AND USES THEREOF**

(71) Applicant: Fundación Centro Nacional de Investigaciones Oncológicas Carlos III, 28029 Madrid (ES)
(72) Inventor: HIDALGO MEDINA, Manuel, E-28029 Madrid (ES); LÓPEZ CASAS, Pedro Pablo, E-28029 Madrid (ES)
(74) Representative: ABG Patentes, S.L.

(57) **Abstract**

The invention relates to an *ex vivo* method for preparing lymphocytes capable of infiltrating a solid tumor in a human subject, comprising: (a) administering CD45+ cells from said subject to an immunodeficient non-human organism, wherein the non-human organism contains a xenograft of the solid tumor generated by implanting cells or tissue from the tumor into the non-human organism; (b) maintaining the non-human organism for sufficient time for the CD45+ cells to infiltrate the tumor xenograft; and (c) recovering lymphocytes from the xenograft. The invention further relates to a cellular population of lymphocytes obtainable by said method and uses thereof.

## Description

### FIELD OF THE INVENTION

The invention relates to the field of adoptive cell transfer therapy and, more in particular, to a method for preparing lymphocytes capable of infiltrating a solid tumor in a human subject, as well as to a cellular population of lymphocytes obtainable by said method and uses thereof

### BACKGROUND ART

Adoptive cell transfer therapy (ACT) refers to the transfer of cells into a patient as a form of cancer immunotherapy. The cells are most commonly derived from the immune system and they are typically altered before being transferred back, with the goal of transferring improved immune functionality. These cells may originate from the patients themselves (autologous cells) or they may come from another individual (allogeneic cells). A specific modality of ACT utilizes tumor infiltrating lymphocytes (TILs) as therapeutic agent. TILs are immune system cells which may present within a tumor mass. These cells are involved in the destruction of cancer cells and their presence in the tumor is usually associated with a better prognosis in the clinical outcome of a patient. One limitation of the use of TILs in ACT therapies is the lack of routine techniques for extraction, purification and expansion of TILs, so that hospitals may have the capability to prepare a cell based TIL product that is effective and safe to be implanted in a patient.

In this regard, US 20140030806 A1 discloses a method for the *in vitro* generation of "young" tumor-infiltrating lymphocytes, where the TILs are between 19 and 35 days old (approximately 3 to 5 weeks). The "young" TIL are then administered to a patient after the patient has received non-myeloablative lymphodepleting therapy in order to induce tumor regression. The TILs are obtained from tumor tissue of patients.

Kawaoka et al. (Oncology Reports: 20, 155-163, 2008) teaches a method of immunotherapy against pancreatic cancer using MUC1-specific cytotoxic T lymphocytes (CTLs). CTLs cells are prepared from PBMC first culturing for 3 days with a human cell line expressing MUC1, and stimulating them subsequently for 7 days with interleukin 2 (IL-2). The CTLs cells are then administered to the patient.

Peiper et al. (Int. J. Cancer: 71, 993-999, 1997) discloses a pancreatic cancer cell line (MPanc-96) established from a sample of pancreatic tissue from a cancer patient, which can be recognized by autologous tumor infiltrating lymphocytes after both *in vivo* and *in vitro* lymphocyte expansion. In the specific case of the *in vivo* expansion, MPanc-96 cells are injected subcutaneously into the flanks of nude mice, specifically ICR-SCID mice, causing the appearance of tumor nodules in the animal. This document shows that invasion of tumor nodules by lymphocytes can be observed when CTLs, obtained from TILs activated with immobilized anti-CD3 and expanded into low-dose IL-2 antibodies, are injected into the mouse.

Meng et al. (J. Immunother. Cancer, 2 (Suppl. 3):P26, 6 November 2014) describes the production of tumor infiltrating lymphocytes from pancreatic cancer tissue and their expansion in a culture medium comprising cytokines IL-2, IL-15 and IL-21. This document announces that TILs obtained by this method will be used in a Phase I clinical trial for use in ACT therapy for patients suffering from pancreatic cancer.

However, the clinical exploitation of the methods described above is limited due to the fact that TILs are obtained as a single batch and used only once in a single course of therapy, particularly when the cells are extracted from a solid tumor. Additionally, TILs cannot always be obtained from a solid tumor, either because the tumor does not naturally contain them, or because the tumor cannot be extracted. The latter situation would correspond to patients who suffer from an advanced stage of the disease.

Thus, there is a need in the art for providing effective methods for the preparation of lymphocytes capable of infiltrating a solid tumor in a human subject.

### SUMMARY OF THE INVENTION

The inventors have identified that immunodeficient non-human organisms wherein the non-human organisms contain a xenograft of a solid tumor from a human subject may be used as means to obtain lymphocytes capable of infiltrating a solid tumor in a human subject suitable for ACT therapy in patients suffering from cancer.

Therefore, in a first aspect, the present invention relates to a method for the *ex vivo* preparation of lymphocytes capable of infiltrating a solid tumor in a human subject, comprising:
(a) administering CD45+ cells from said subject to an immunodeficient non-human organism, wherein the non-human organism contains a xenograft of said solid tumor generated by implanting cells or tissue from the tumor into the non-human organism;
(b) maintaining the non-human organism for sufficient time for the CD45+ cells to infiltrate the tumor xenograft; and
(c) recovering lymphocytes from the xenograft.

In a second aspect, the invention relates to a cellular population of lymphocytes obtainable by the method described above.

In a third aspect, the invention relates to a pharmaceutical composition comprising the cellular population of lymphocytes obtainable by the method described above and a pharmaceutically acceptable vehicle.

In a fourth aspect, the present invention relates to the cellular population or the pharmaceutical composition described above for use in medicine.

Finally, in a fifth aspect, the invention relates to the cellular population or the pharmaceutical composition described above for use in the treatment of a subject suffering from cancer.

### DETAILED DESCRIPTION OF THE INVENTION

### A method for the ex vivo preparation of lymphocytes capable of infiltrating a solid tumor from a human subject

The inventors have identified that immunodeficient non-human organisms containing a xenograft of a solid tumor from a human subject may be used as means to obtain lymphocytes capable of infiltrating a solid tumor in the human subject suitable for ACT therapy in patients suffering from cancer.

Therefore, in a first aspect, the present invention relates to a method for the *ex vivo* preparation of lymphocytes capable of infiltrating a solid tumor in a human subject, comprising:
(a) administering CD45+ cells from said subject to an immunodeficient non-human organism, wherein the non-human organism contains a xenograft of said solid tumor generated by implanting cells or tissue from the tumor into the non-human organism;
(b) maintaining the non-human organism for sufficient time for the CD45+ cells to infiltrate the tumor xenograft; and
(c) recovering lymphocytes from the xenograft.

The term "lymphocyte", as used herein, refers to any of the subtypes of white blood cell in an organism's immune system. Lymphocytes may include natural killer cells (NK cells), T cells, and B cells. In a preferred embodiment of the invention the lymphocyte is any tumor infiltrating cell with immune activity.

The term "tumor" or "cancer", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. In a preferred embodiment, the cancer appears as a benign tumor, i.e. tumors that cannot spread by invasion or metastasis, i.e., they only grow locally. In another embodiment, the cancer appears as a malign tumor, i.e. a tumor that is capable of spreading by invasion and metastasis.

In a preferred embodiment of the invention, the solid tumor is any solid tumor for which TILs can be produced and which can be transferred to a non-human animal to give rise to a xenograft. Non-limiting examples of cancers include acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the vulva, chronic lymphocytic leukemia, chronic myeloid cancer, cervical cancer, glioma, Hodgkin lymphoma, hypopharynx cancer, kidney cancer, larynx cancer, liver cancer, lung cancer, malignant mesothelioma, melanoma, multiple myeloma, nasopharynx cancer, non- Hodgkin lymphoma, ovarian cancer, peritoneum, omentum, and mesentery cancer, pharynx cancer, prostate cancer, rectal cancer, renal cancer, skin cancer, soft tissue cancer, testicular cancer, thyroid cancer, ureter cancer, urinary bladder cancer, and digestive tract cancer such as, e.g., esophageal cancer, gastric cancer, pancreatic cancer, stomach cancer, small intestine cancer, gastrointestinal carcinoid tumor, cancer of the oral cavity, colorectal cancer, and hepatobiliary cancer

The cancer can refer to the primary location of a cancer as well as a metastatic and/or a recurrent location.

The term "metastasis" refers to the spread of malignant tumor cells from one organ or part to another non-adjacent organ or part. Cancer cells can "break away," "leak," or "spill" from a primary tumor, enter lymphatic and blood vessels, circulate through the bloodstream, and settle down to grow within normal tissues elsewhere in the body. When tumor cells metastasize, the new tumor is called a secondary or metastatic cancer or tumor.

As used herein, the term "subject" or "patient" in the present invention is understood as a female or male human being of any race or age. In the context of the present invention, the subject is a subject who potentially suffers from cancer or a disease associated with inflammation or a subject who has been previously diagnosed with cancer or a disease associated with inflammation. In a preferred embodiment, the subject has not been treated with chemotherapy or radiotherapy prior to the extraction of a tumor tissue sample. In yet another embodiment, the patient has not undergone surgical resection of the tumor.

The term "capable of infiltrating a solid tumor", as used herein, refers to a lymphocyte which has the capacity of leaving the blood stream and migrating into the tumor.

In a first step, the method for the ex vivo preparation of lymphocytes according to the present invention comprises administering CD45+ cells from the patient to an immunodeficient non-human organism, wherein the non-human organism contains a xenograft of said solid tumor generated by implanting cells or tissue from the tumor into the non-human organism.

The term "CD45+ cells", as used herein refers to the fraction from the peripheral blood monocyte cell population (PBMC) which is positive for the cell surface marker CD45 (also known as lymphocyte common antigen). It will be understood that the population of CD45+ cells which is administered to the non-human organism in the first step of the method need not be comprises exclusively of CD45+ cells and that other peripheral blood cells may also be present in the preparation. The preparation of CD45+ cells for use in the first step of the method according to the present invention may contain at least 90%, at least 92%, at least 94%, at least 96%, at least 98%, at least 99%, at least 99,5%, at least 99,6%, at least 99,7%, at least 99,8%, at least 99,9% or more CD45+ cells.

As used herein, the term "immunodeficient", when referred to the non-human organism used in the first step of the method according to the present invention, refers to an animal's impaired or otherwise not fully functioning immune system, for example an inability to produce a normal amount of B-cells, T-cells, NK-cells, etc. The immunodeficient phenotype can be, in one embodiment, a result of a naturally occurring genetic defect, or, in another embodiment, a result of an induced genetic defect. Immunodeficiency may be produced by, for example, but not limited to, mutations, irradiation, a chemical or pharmaceutical, or a virus. Examples of immunodeficient mice include nude (nu⁻nu-⁾ mice, nude and severe combined immunodeficiency (SCID) mice, non-obese diabetic (NOD) mice, NOD/SCID mice, NSG (NOD/SCID/γc^{-/-}) mice, Non-Obese Diabetic (NOD) Shi-Scid IL-2R γ^{null} (NOG) mice, Rag-1 (rag-r^{1/1}/γc^{-/-}) mice, or Rag-2(rag-2^{-/-}/gc^{-/-}) BRG mice (BALB/c-Rag2^{null}/IL2rγ^{null}), Rag 1^{-/-} mice, Rag Rag 1^{-/-} /γc^{-/-} mice, Scid mouse, NOD/Shi mouse, IL-2R gamma^{null} mouse, NOD/Sci-Scid mouse.

In a preferred embodiment, the immunodeficient mice are NOD mice carrying various mutations in the interleukin- 2 receptor gamma chain (IL2λ) gene. Examples of such mice include NOD/SCID IL2r^{null} and NOD/SCID IL2rγ^{Trunc} mice.

In a preferred embodiment of the invention, the immunodeficient non-human organism lacks T and B lymphocytes and Natural Killer (NK) cells.

The term "non-human organism", as used herein, refers to all animals classified as mammals except for humans and includes, but is not restricted to, domestic and farm animals, primates, cows, horses, pigs, sheep, goats, dogs, cats, or rodents. In a preferred embodiment of the invention, the immunodeficient non-human organism is a rodent. In a more preferred embodiment of the invention, the immunodeficient non-human organism is a mouse. In a still more preferred embodiment of the invention, the immunodeficient non-human organism is a NOD/SCID/IL2λ receptor null (NSG) mouse. The term "NOD/SCID/IL2λ-receptor null (NSG) mouse" refers to laboratory generated strain of mice that lack mature T cells, B cells, and natural killer (NK) cells. NSG mice are also deficient in multiple cytokine signaling pathways and they have many defects in innate immunity. These deficiencies allow for the engraftment of a wide range of primary human cells (Shultz et al., 2007, Nat. Rev. Immunol. 7:118-30). As used herein, the term "xenograft" refers to the material obtained after transplanting living cells, tissues or organs from one species to another. In the context of the present invention, it specifically refers to a transplant of living cells or tissue from a human to a non-human organism, preferably an immunodeficient non-human organism, more preferably a NOD/SCID/IL2λ receptor null (NSG) mouse.

In a particular embodiment, the xenograft is implanted in the non-human organism in the vicinity of the organ in which the tumor is found in the human subject. In another embodiment, the xenograft is implanted in the non-human organism in the organ in which the tumor is found in the human subject. In another embodiment, the xenograft is implanted in the non-human organism subcutaneously.

In a preferred embodiment of the invention, the CD45+ cells are administered directly into the xenograft.

In a second step, the method of the invention comprises maintaining the non-human organism for sufficient time for the CD45+ cells to infiltrate the tumor xenograft. In a preferred embodiment, the non-human organism is maintained for at least 3, 4, 5, 6, 7, 8, 9, or 10 weeks before the lymphocytes are recovered from the non-human organism. It will be understood that not all types of the CD45+ cells administered in the first step will infiltrate the tumor. Instead, infiltration into the tumor will be limited to the lymphocytes found in the CD45+ cell population.

During this second step, the non-human organism is maintained for at least 3, 4, 5, 6, 7, 8, 9, or 10 weeks so that said CD45+ may infiltrate the tumor.

As the xenograft becomes populated with lymphocytes capable of infiltrating the human tumor, said lymphocytes react cytotoxically against the tumor and become activated.

The term "activation", as used herein, refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. In some embodiments, activated T cells are characterized by the transient expression of characteristic surface molecules such as the CD40 ligand, 4-1BB, CD69 and/or CD25.

In a third step, the method according to the invention, comprises recovering lymphocytes from the xenograft.

The recovery step can be carried out using any standard technique known in the art for the recovery of infiltrating lymphocytes. In a preferred embodiment, the tumor xenograft is first surgically excised from the nonhuman organism. The resected tumor is treated in order to obtain a single cell suspension. The single cell suspension can be obtained in any suitable manner, e.g., mechanically (disaggregating the tumor using, e.g., a gentleMACS(TM) Dissociator, Miltenyi Biotec, Auburn, Calif.) or enzymatically (e.g., collagenase, hyaluronidase, DNase or a combination thereof).

This step facilitates further processing of the tumor sample and separation of the different cells types found in the tumor.

In one embodiment, the lymphocytes infiltrating the tumor xenograft are isolated using antibodies specific for one or more markers specific for the lymphocytes which have infiltrated the tumor. In another embodiment, the lymphocytes infiltrating the tumor xenograft are isolated by buoyant density centrifugation, for instance using a discontinuous (75%/100%) Ficoll-Hypaque gradient at 550g for 15 h. Using this system, the lymphocytes can be recovered substantially free from tumor cells from the the lower interface. In another embodiment, the lymphocytes infiltrating the tumor xenograft are isolated as described in Meng Q et al., J. Immunother., 39: 81-9, 2016.

In yet a preferred embodiment, the method of the invention further comprises the purification, expansion and/or activation of the lymphocytes obtained in step (c). These lymphocytes capable of infiltrating a solid tumor in a human subject obtained by the method described above may be extracted and expanded using known methods well known in the art (Meng Q, et al. J. Immunother. 39: 81-9, 2016.).

Expansion of lymphocytes, including tumor-infiltrating lymphocytes, such as T cells can be accomplished by any of a number of methods as are known in the art. For example, T cells can be rapidly expanded using non-specific T-cell receptor stimulation in the presence of feeder lymphocytes and interleukin-2 (IL-2), IL-7, IL-15, IL-21, or combinations thereof.

In one embodiment, the lymphocytes obtained by the method according to the present invention can be cultured in the presence of an immune cell stimulating ligand, such as at least one of anti-CD3 antibody and/or anti-CD28 antibody. In one embodiment, the lymphocytes cells can be cultured in the presence of anti-CD3 antibody and/or anti-CD28 antibody and in IL-2. In another embodiment, the anti-CD3 and/or anti-CD8 antibodies are bound to beads that are cultured with the lymphocytes obtained by the method according to the present invention. The anti-CD3/CD28 antibodies may be immobilized on the beads, either on the same bead, i.e., "cis," or to separate beads, i.e., "trans." By way of example, the agent providing the primary activation signal is an anti-CD3 antibody or an antigen-binding fragment thereof and the agent providing the co-stimulatory signal is an anti-CD28 antibody or antigen- binding fragment thereof; and both agents are co-immobilized to the same bead in equivalent molecular amounts. In one embodiment, a 1: 1 ratio of each antibody bound to the beads for CD 137+ cell expansion and cell growth is used. In certain aspects of the present invention, a ratio of anti CD3:CD28 antibodies bound to the beads is used such that an increase in cell expansion is observed as compared to the expansion observed using a ratio of 1: 1. In one particular embodiment an increase of from about 1 to about 3 fold is observed as compared to the expansion observed using a ratio of 1: 1. In one embodiment, the ratio of CD3:CD28 antibody bound to the beads ranges from 100: 1 to 1: 100 and all integer values there between. In one aspect of the present invention, more anti-CD28 antibody is bound to the particles than anti-CD3 antibody, i.e., the ratio of CD3:CD28 is less than one. In certain embodiments of the invention, the ratio of anti CD28 antibody to anti CD3 antibody bound to the beads is greater than 2: 1. In one particular embodiment, a 1: 100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1: 10 CD3:CD28 ratio of antibody bound to beads is used. In another embodiment, a 1:3 CD3:CD28 ratio of antibody bound to the beads is used. In yet another embodiment, a 3: 1 CD3:CD28 ratio of antibody bound to the beads is used.

In another embodiment, the lymphocytes isolated according to the present invention are selectively isolated and co-cultured with HLA-matched tumor cell lines. Examples of HLA- matched tumor cell lines may include allogeneic cancer cell lines (Dudley et al., 2003, J Immunother., 26: 332-42), HLA-matched melanoma cell lines, autologous cancer cells, and any other cells that are HLA-matched to the lymphocytes.

In one embodiment of the present invention, the mixture may be cultured for several hours (about 3 hours) to about 14 days or any hourly integer value in between. In another embodiment, the mixture may be cultured for 21 days. In one embodiment of the invention the lymphocytes are cultured for about seven days.

Conditions appropriate for cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-gamma, IL-4, IL-7, GM-CSF, IL-10, IL-12, IL-15, TGFp, and TNF-a or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, a- MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37 degrees C) and atmosphere (e.g., air plus 5 percent C(¾).

The TIL obtained according to the method of the present invention can be tested by any method known in the art, e.g., by measuring cytokine release (e.g., interferon-gamma) following co-culture with tumor cells. In one embodiment, the autologous ACT method comprises enriching cultured TILs for CD8+ T cells prior to rapid expansion of the cells. Following culture of the TILs in IL-2, the T cells are depleted of CD4+ cells and enriched for CD8+ cells using, for example, a CD8 microbead separation (e.g., using a CliniMACS CD8 microbead system (Miltenyi Biotec)). In some embodiments, a T-cell growth factor that promotes the growth and activation of the autologous T cells is administered to the mammal either concomitantly with the autologous T cells or subsequently to the autologous T cells. The T-cell growth factor can be any suitable growth factor that promotes the growth and activation of the autologous T-cells.

Examples of suitable T-cell growth factors include interleukin (IL)-2, IL-7, IL-15, IL-12 and IL- 21, which can be used alone or in various combinations, such as IL-2 and IL-7, IL-2 and IL-15, IL-7 and IL-15, IL-2, IL-7 and IL-15, IL-12 and IL-7, IL- 12 and IL-15, or IL-12 and IL2.

As it would be obvious to a person skilled in the art, the tumors from a first-generation of non-human organisms carrying the xenograft can be extracted, divided into pieces, and implanted again in multiple non-human organisms ("second generation"). This process can be repeated several times to obtain a large number of non-human organisms carrying a xenograft from a single patient. As a consequence and in contrast with conventional methods, a cell therapy product based on lymphocytes capable of infiltrating a solid tumor in a human subject obtained by the method of the present invention may be prepared as many times as necessary. Repeated treatment with these lymphocytes is thus possible.

### Cellular population of lymphocytes capable of infiltrating a solid tumor in a human subject

In a second aspect, the invention relates to a cellular population of lymphocytes obtainable by the method described above.

The cellular population according to the present invention includes the of lymphocytes capable of infiltrating a solid tumor isolated according to the present invention, as well as the cell populations resulting from the expansion and/or activation of the initial cell population.

In one embodiment, the lymphocytes are T-cells which are specific towards the tumor from which the xenograft was generated.

The term "tumor-specific T cell", as used herein, refers to a T cell which is capable of mounting a cytotoxic reaction against a specific tumor, usually due to the presence in the tumor of a tumor-associated antigen (TAA) which is capable of stimulating the T cell. In a preferred embodiment, the tumor-specific T cell is a cytotoxic T cell. Methods for determining whether the T-cells are activated are widely available and known to the person skilled in the art. In a preferred embodiment of the invention, the activated lymphocytes are defined by their reactivity in a standard assay by interferon gamma (IFNγ) production as described elsewhere (Meng Q, et al. J. Immunother. 39: 81-9, 2016.).

All the terms and embodiments previously described are equally applicable to this aspect of the invention.

### A pharmaceutical composition comprising a cellular population of lymphocytes capable of infiltrating a solid tumor in a human subject and a pharmaceutically acceptable vehicle

In a third aspect, the invention relates to a pharmaceutical composition comprising the cellular population described above having therapeutic activity or susceptible of being used with therapeutic purposes, and a pharmaceutically acceptable vehicle.

The pharmaceutical composition according to the present invention comprises the cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

Suitable doses of cells for a therapeutic effect would be at least 10⁵ or between about 10⁵ and about 10¹⁰ cells per dose, for example, preferably in a series of dosing cycles. An exemplary dosing regimen consists of four one-week dosing cycles of escalating doses, starting at least at about 10⁵ cells on Day 0, for example increasing incrementally up to a target dose of about 10¹⁰ cells within several weeks of initiating an intra-patient dose escalation scheme.

Preferably, lymphocytes produced by the disclosed methods are administered as an intra-arterial or intravenous infusion, which preferably lasts about 30 to about 60 minutes. Other examples of routes of administration include intraperitoneal, intrathecal, intralymphatic, subcutaneous, intracavitary (for example by reservoir-access device), intraperitoneal, and direct injection into a tumor mass.

The pharmaceutical compositions contain highly pure populations of the lymphocytes according to the invention (greater than 70 percent, 80 percent, 90 percent or more of the cells).

All the terms and embodiments previously described are equally applicable to this aspect of the invention.

### Uses of lymphocytes capable of infiltrating a solid tumor in a human subject for adoptive cell transfer

In a fourth aspect, the present invention relates to the cellular population or the pharmaceutical composition described previously for use in medicine.

In a fifth aspect, the invention also relates to the cellular population or the pharmaceutical composition described previously for use in the treatment of a subject suffering from cancer.

The term "therapy" or "treatment", as used herein, refers to the attempted remediation of a health problem, usually following a diagnosis. As such, it is not necessarily a cure, i.e. a complete reversion of a disease. Said therapy may or may not be known to have a positive effect on pancreatic cancer or, in other words, to be useful to treat pancreatic cancer. Therapies include, without limitation, surgery, radiotherapy, chemotherapy, or targeted therapies (e.g. using antibodies) and the choice of a therapy or a combination of therapies depends, for example, on the stage of the cancer, whether the cancer has recurred and the patient's general health.

The ACT methods according to the present invention can be used for the treatment of any type of solid tumor. Suitable tumors that can be treated using the cellular population or the pharmaceutical composition described are as defined above.

In a preferred embodiment of the invention, the solid tumor is acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the vulva, chronic lymphocytic leukemia, chronic myeloid cancer, cervical cancer, glioma, Hodgkin lymphoma, hypopharynx cancer, kidney cancer, larynx cancer, liver cancer, lung cancer, malignant mesothelioma, melanoma, multiple myeloma, nasopharynx cancer, non- Hodgkin lymphoma, ovarian cancer, peritoneum, omentum, and mesentery cancer, pharynx cancer, prostate cancer, rectal cancer, renal cancer, skin cancer, soft tissue cancer, testicular cancer, thyroid cancer, ureter cancer, urinary bladder cancer, and digestive tract cancer such as, e.g., esophageal cancer, gastric cancer, pancreatic cancer, stomach cancer, small intestine cancer, gastrointestinal carcinoid tumor, cancer of the oral cavity, colorectal cancer, and hepatobiliary cancer.

In a preferred embodiment, the cell population according to the present invention is autologous with respect to the cancer to be treated, i.e. the cell population is used for the treatment of the cancer which was used to obtain the xenograft in the non-human organism from with the cellular population was isolated. In another embodiment, the cell population according to the present invention is allogeneic with respect to the cancer to be treated, i.e. the cell population is used for the treatment of a cancer in a subject, wherein the cancer is of the same type as that which was used to obtain the xenograft in the non-human organism but wherein the original cancer is found in a different subject from tge subject which is to be treated. In one embodiment, the cell population according to the present invention is used for the treatment of a cancer which is of the same type as the cancer from which the xenograft was originally generated, i.e. the cell population is isolated from a xenograft obtained from a pancreatic tumor and is used for the treatment of pancreatic cancer. In another embodiment, the cell population according to the present invention is used for the treatment of a cancer which is of a different type as the cancer from which the xenograft was originally generated, i.e. the cell population is isolated from a xenograft obtained from a pancreatic tumor and is used for the treatment of cancer which is not pancreatic. In another embodiment, the cell population according to the present invention is used for the treatment of a cancer which is a metastasis from a primary tumor which is of the same type as the cancer from which the xenograft was originally derived, i.e. i.e. the cell population is isolated from a xenograft obtained from a pancreatic tumor and is used for the treatment of metastasis derived from a pancreatic cancer. In another embodiment, the cell population according to the present invention is used for the treatment of a cancer which is a metastasis from a primary tumor which is of a different type as the cancer from which the xenograft was originally derived, i.e. i.e. the cell population is isolated from a xenograft obtained from a pancreatic tumor and is used for the treatment of metastasis derived from a primary tumor which is not pancreatic cancer.

In a preferred embodiment, the cancer is pancreatic cancer. In another embodiment t, the tumor to be treated does not contain infiltrating lymphocytes. In another embodiment, the cancer is metastatic cancer.

Since the method for obtaining lymphocytes capable of infiltrating a tumor according to the present invention result in the activation of the lymphocytes within the xenograft, the lymphocytes can be used in an adoptive cell therapy according to the present invention without a prior activation step. Accordingly, in one embodiment, the lymphocytes for use according to the present invention are used directly without a prior activation step.

In some embodiments, non-myeloablative lymphodepleting chemotherapy is administered to the mammal prior to administering to the mammal the expanded tumor-infiltrating lymphocytes. The purpose of lymphodepletion is to make room for the infused lymphocytes, in particular by eliminating regulatory T cells and other non-specific T cells which compete for homeostatic cytokines. Non-myeloablative lymphodepleting chemotherapy can be any suitable such therapy, which can be administered by any suitable route known to a person of skill. The non-myeloablative lymphodepleting chemotherapy can comprise, for example, the administration of cyclophosphamide and fludarabine, particularly if the cancer is melanoma, which can be metastatic. A preferred route of administering cyclophosphamide and fludarabine is intravenously. Likewise, any suitable dose of cyclophosphamide and fludarabine can be administered. Preferably, around 40-80 mg/kg, such as around 60 mg/kg of cyclophosphamide is administered for approximately two days after which around 15-35 mg/m², such as around 25 mg/m² fludarabine is administered for around five days, particularly if the cancer is melanoma.

Preferably, expanded lymphocytes produced by the disclosed methods are administered as an intra-arterial or intravenous infusion, which preferably lasts about 30 to about 60 minutes. Other examples of routes of administration include intraperitoneal, intrathecal and intralymphatic. Likewise, any suitable dose of lymphocytes can be administered. In one embodiment, about 1 x 10¹⁰ lymphocytes to about 15 x 10¹⁰ lymphocytes are administered.

In a particular embodiment, the invention relates to the cellular population or the pharmaceutical composition for use as described above wherein the patient is preconditioned by anti-tumor therapy prior to the administration of the cellular population of tumor infiltrating lymphocytes or pharmaceutical composition. In a preferred embodiment, the anti-tumor therapy comprises radiotherapy, immunotherapy, chemotherapy, or a combination thereof.

The term "chemotherapy", as used herein, refers to a treatment that at least partially inhibits the development or progression of a cancer, including inhibiting in whole or in part symptoms associated with the cancer.

The term "immunotherapy" refers to a therapeutic approach for treating cancer in which immune cells are administered to a host with the aim that the cells mediate either directly or indirectly specific immunity to (i.e., mount an immune response directed against) the undesired cells. In preferred embodiments, the immune response results in inhibition of tumor and/or metastatic cell growth and/or proliferation and most preferably results in neoplastic cell death and/or resorption. The immune cells can be derived from a different organism/host (exogenous immune cells) or can be cells obtained from the subject organism (autologous immune cells). There are two distinct types of immunotherapy: passive immunotherapy uses components of the immune system to direct targeted cytotoxic activity against cancer cells, without necessarily initiating an immune response in the patient, while active immunotherapy actively triggers an endogenous immune response. Passive strategies include the use of the monoclonal antibodies (mAbs) produced by B cells in response to a specific antigen. The development of hybridoma technology in the 1970s and the identification of tumor-specific antigens permitted the pharmaceutical development of mAbs that could specifically target tumor cells for destruction by the immune system. Thus far, mAbs have been the biggest success story for immunotherapy; the top three best-selling anticancer drugs in 2012 were mAbs. Among them is rituximab (Rituxan, Genentech), which binds to the CD20 protein that is highly expressed on the surface of B cell malignancies such as non-Hodgkin's lymphoma (NHL). Rituximab is approved by the FDA for the treatment of NHL and chronic lymphocytic leukemia (CLL) in combination with chemotherapy. Another important mAb is trastuzumab (Herceptin; Genentech), which revolutionized the treatment of HER2 (human epidermal growth factor receptor 2)-positive breast cancer by targeting the expression of HER2.

In order to actively drive an antitumor immune response, therapeutic cancer vaccines have been developed. Unlike the prophylactic vaccines that are used preventatively to treat infectious diseases, therapeutic vaccines are designed to treat established cancer by stimulating an immune response against a specific tumor-associated antigen. In 2010, sipuleucel- T (Provenge; Dendreon Corporation) was approved by the FDA for the treatment of metastatic, castration-resistant prostate cancer based on the results of the IMPACT (Immunotherapy Prostate Adenocarcinoma Treatment) trial in which it improved OS by 4.1 months and reduced the risk of death by 22 percent versus placebo. The advantage of active immunotherapies is that they have the potential to provide long-lasting anticancer activity by engaging both the innate and adaptive arms of the immune response. While mAbs are typically considered passive immunotherapies, there is increasing evidence that they also induce an adaptive immune response via a "vaccination-like" effect.

Generating optimal "killer" CD8 T cell responses also requires T cell receptor activation plus co-stimulation, which can be provided through ligation of tumor necrosis factor receptor family members, including OX40 (CD 134) and 4- IBB (CD 137). OX40 is of particular interest as treatment with an activating (agonist) anti-OX40 mAb augments T cell differentiation and cytolytic function leading to enhanced anti-tumor immunity against a variety of tumors.

The term "radiotherapy" or "radiotherapeutic treatment" is a term commonly used in the art to refer to multiple types of radiation therapy including internal and external radiation therapies or radio immunotherapy, and the use of various types of radiations including X-rays, gamma rays, alpha particles, beta particles, photons, electrons, neutrons, radioisotopes, and other forms of ionizing radiations.

In another particular embodiment, the invention relates to the cellular population or the pharmaceutical composition for use as described above wherein the chemotherapy comprises an alkylating agent, preferably cyclophosphamide, an antimetabolite, preferably fludarabine, or a combination thereof, or wherein the immunotherapy comprises immune check-point inhibitors.

Examples of alkylating agents include chlorambucil, chlormethine, cyclophosphamide, ifosphamide, melphalan, carmustine, fotemustine, lomustine, streptozocin, carboplatin, cisplatin, oxaliplatin, satraplatin, busulfan, dacarbazine, procarbazine, temozolomide, thioTEPA, treosulfan, and uramustine.

The term "antimetabolite", as used herein, relates, in a broad sense, to substances which disturb normal metabolism and substances which inhibit the electron transfer system to prevent the production of energy-rich intermediates, due to their structural or functional similarities to metabolites that are important for living organisms (such as vitamins, coenzymes, amino acids and saccharides). Examples of antimetabolites that have antitumor activities include but are not limited to folic acid analogs (e.g., methotrexate (amethopterin)), denopterin, edatrexate, methotrexate, nolatrexed, pemetrexed, piritrexim, pteropterin, raltitrexed, trimetrexate; pyrimidine analogs (e.g., fluorouracil (5- fluorouracil; 5-FU(R)), floxuridine (fluorode-oxyuridine; FudR), doxifluridine and cytarabine (cytosine arabinoside)); purine analogs (e.g., fludarabine (2-fluoroadenine), mercaptopurine (6-mercaptopurine; 6-MP), thioguanine (6-thioguanine; TG), and pentostatin (2'-deoxycoformycin)).

The cellular population of tumor infiltrating lymphocytes for use in the treatment of a subject suffering from cancer as described above is preferably administered in combination with cytokines.

The term "cytokine" refers to small soluble proteins secreted by cells that can alter the behavior or properties of the secreting cell or another cell. Cytokines bind to cytokine receptors and trigger a behavior or property within the cell, for example, cell proliferation, death or differentiation. Exemplary cytokines include, but are not limited to, interleukins (e.g., IL-2, IL-3, IL-4, IL-5, IL-6, IL-7, IL-8, IL-9, IL-10, IL-11, IL-12, IL-13, IL-15, IL-16, IL-17, IL-18, IL-la, IL-1 beta, and IL-1 RA), granulocyte colony stimulating factor (G-CSF), granulocyte-macrophage colony stimulating factor (GM-CSF), oncostatin M, erythropoietin, leukemia inhibitory factor (LIF), interferons, B7.1 (also known as CD80), B7.2 (also known as B70, CD86), TNF family members (TNF-alpha, TNF- beta, LT- beta, CD40 ligand, Fas ligand, CD27 ligand, CD30 ligand, 4-1BBL, Trail), and MIF. The cytokine levels can be determined by several methods well-known in the art, such as ELISA, ELISPOT, Western Blot, and flow cytometry.

In a preferred embodiment of the present invention, the cytokine is IL-2.

In another embodiment, the cellular population or the pharmaceutical composition for use in the treatment of a subject suffering from cancer as described above is applied to a tumor that does not contain infiltrating lymphocytes.

In another particular embodiment, the invention relates to the cellular population or the pharmaceutical composition for use in the treatment of a subject suffering from cancer as described above where the cancer to be treated is metastatic cancer.

The term "metastasis" is understood as the distance propagation, fundamentally by the lymphatic or blood stream, of the cancer causing cells, and the growth of new tumors in the destination sites of said metastasis.

All the terms and embodiments previously described are equally applicable to these aspects of the invention.

### Method for the ex vivo activation of lymphocytes capable of infiltrating a solid tumor from a human subject

The authors of the present invention have shown that the lymphocytes capable of infiltrating the human tumor obtained according to the method of the present invention populate the xenograft and become activated within the xenograft. This a clear advantage over methods for adoptive cell therapy known in the art, wherein the lymphocytes directly obtained from the patient are not capable of infiltrating a human tumor if they are not first subjected to an activation step. Thus, according to the method of the invention, lymphocytes isolated from the xenograft can be extracted and used directly without the need of an activation step.

In a sixth aspect, the present invention relates to a method for the *ex vivo* activation of lymphocytes capable of infiltrating a solid tumor in a human subject, comprising:
(a) administering lymphocytes from said subject to an immunodeficient non-human organism, wherein the non-human organism contains a xenograft of said solid tumor generated by implanting cells or tissue from the tumor into the non-human organism and
(b) maintaining the non-human organism for sufficient time for the lymphocytes to infiltrate the tumor xenograft and become activated.

The term "activation", as used herein, refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. In some embodiments, activated T cells are characterized by the transient expression of characteristic surface molecules such as the CD40 ligand, 4-1BB, CD69 and/or CD25.

In a first aspect, the method for the activation of lymphocytes lymphocytes capable of infiltrating a solid tumor comprises administering lymphocytes from said subject to an immunodeficient non-human organism, wherein the non-human organism contains a xenograft of said solid tumor generated by implanting cells or tissue from the tumor into the non-human organism. All the terms and embodiments previously described are equally applicable to this aspect of the invention.

In a second step, the method for the activation of lymphocytes lymphocytes capable of infiltrating a solid tumor comprises maintaining the non-human organism for sufficient time for the lymphocytes to infiltrate the tumor xenograft and become activated.

In a preferred embodiment, the lymphocytes to be activated are non-activated TILs obtained directly from a patient. In another embodiment, the lymphocytes to be activated are found within a population of CD45+ cells isolated from the patient.

All the terms and embodiments previously described are equally applicable to this aspect of the invention.

The invention refers to the following aspects:
1. A method for the *ex vivo* preparation of lymphocytes capable of infiltrating a solid tumor in a human subject, comprising:
   (a) administering CD45+ cells from said subject to an immunodeficient non-human organism, wherein the non-human organism contains a xenograft of said solid tumor generated by implanting cells or tissue from the tumor into the non-human organism;
   (b) maintaining the non-human organism for sufficient time for the CD45+ cells to infiltrate the tumor xenograft; and
   (c) recovering lymphocytes from the xenograft.
2. The method of aspect 1, wherein the solid tumor is selected from the group consisting of lung, genitourinary, gynecologic, breast, head and neck, central nervous system, kidney, pancreatic, and any other gastrointestinal tumor.
3. The method of any of aspects 1 or 2, wherein the immunodeficient non-human organism lacks T and B lymphocytes and Natural Killer (NK) cells.
4. The method of any of aspects 1 to 3, wherein the immunodeficient non-human organism is a NOD/SCID/IL2λ receptor null (NSG) mouse.
5. The method of any of aspects 1 to 4 wherein the xenograft is implanted in the non-human organism in the vicinity of the organ in which the tumor is found in the human subject, in of the organ in which the tumor is found in the human subject or subcutaneously.
6. The method of any of aspects 1 to 5, wherein the recovery step (c) is performed at least 3 weeks after the administration step (a).
7. The method of any of aspects 1 to 6 further comprising the purification, expansion and/or activation of the lymphocytes obtained in step (c).
8. The method of aspect 7 wherein the expansion of the lymphocytes is carried out by stimulating the lymphocytes with a cytokine combination comprising IL-2, IL-15, and IL-21
9. The method of aspects 7 or 8 wherein the activation of the lymphocytes is carried using an anti-CD3 antibody in the presence of irradiated feeder cells.
10. A cellular population of lymphocytes obtainable by the method of any of aspects 1 to 9.
11. The cellular population of aspect 10, wherein the lymphocytes are tumor-specific T cells.
12. A pharmaceutical composition comprising the population of any of aspects 10 or 11 and a pharmaceutically acceptable vehicle.
13. The cellular population of any of aspects 10 or 11 or the pharmaceutical composition of aspect 12 for use in medicine.
14. The cellular population of any of aspects 10 or 11 or the pharmaceutical composition of aspect 12 for use in the treatment of a subject suffering from cancer.
15. The cellular population or the pharmaceutical composition for use according to aspect 14 wherein the cancer is pancreatic cancer.
16. The cellular population or the pharmaceutical composition for use according to aspect 15 wherein the patient is preconditioned by anti-tumor therapy prior to the administration of the cellular population of tumor infiltrating lymphocytes or pharmaceutical composition.
17. The cellular population or the pharmaceutical composition for use according to aspect 16 wherein the anti-tumor therapy comprises radiotherapy, immunotherapy, chemotherapy, or a combination thereof.
18. The cellular population or the pharmaceutical composition for use according to aspect 17 wherein the chemotherapy comprises an alkylating agent, preferably cyclophosphamide, an antimetabolite, preferably fludarabine, or a combination thereof, or wherein the immunotherapy comprises immune check-point inhibitors.
19. The cellular population or the pharmaceutical composition for use according to any of aspects 14 to 18 wherein the administration of the cellular population of tumor infiltrating lymphocytes is administered in combination with cytokines.
20. The cellular population or the pharmaceutical composition for use according to any of aspects 14 to 19, wherein the tumor to be treated does not contain infiltrating lymphocytes.
21. The cellular population or the pharmaceutical composition for use according to any of aspects 14 to 20, wherein the cancer is metastatic cancer.
22. A method for the *ex vivo* activation of lymphocytes against a tumor found in subject, comprising:
   (a) administering CD45+ cells from said subject to an immunodeficient non-human organism, wherein the non-human organism contains a xenograft of said solid tumor generated by implanting cells or tissue from the tumor into the non-human organism and
   (b) maintaining the non-human organism for sufficient time for the lymphocytes present in the CD45+ cells to infiltrate the tumor xenograft and to be activated.

The invention is hereby described by way of the following examples, which are to be construed as merely illustrative and not limitative of the scope of the invention.

### EXAMPLES

### Example 1: Method to obtain lymphocytes capable of infiltrating a solid tumor

The inventors disclose in this specific example a methodology to generate personalized autologous TILs using xenograft mice. The process comprises the following steps:

### 1. Venerating of xenografted mice from a patient tumor.

The tumor is implanted subcutaneously in the mouse pancreas according to published and conventional methods as described elsewhere (Hidalgo et al,, Mol. Cancer Ther. 2011, 10:1311-6).

Pretreatment: all animal-related work is done on aseptic conditions. For general anesthesia, the inventors use the Viking Medical Vaporizer, which combines oxygen and isoflurane into an anesthetic gas. After recovery, mice are monitored daily for deterioration until healing of incision, and euthanized by CO2 inhalation in case of signs for lethargy, cachexia, or weight loss.

A freshly collected tumor from a perpetuated xenograft (derived from a patient) is cut into small pieces of 1-2 mm³ and embedded in a matrigel solution (Cat. No. 05850; BD). Six-week-old Nod/SCID/IL2λ-receptor null (NSG) mice are anesthetized using isoflurane. After the abdominal skin is sterilized, an incision is made in the upper left abdomen, and the pancreas (under the spleen) is exposed. Tumor pieces from the perpetuated xenograft are placed on the pancreatic tissue using 6-0 mm absorbable suture. Tumor growth is monitored by ultrasound.

Mice are monitored during the whole study and they are sacrificed when any of the following symptoms appear: unacceptable increased tumor size, loss of body weight, lethargy, respiratory insufficiency and/or pain.

### 2. Administration of CD45 + blood cells from same patient.

Patient peripheral blood (30 mL) is collected in EDTA tubes and red blood cells are lysed using erythrocyte lysis solution (Cat.No:79217; Qiagen) for 5 min at room temperature. The peripheral blood mononuclear cells (PBMC) are magnetically labeled with CD45 MicroBeads (Cat. No: 130-045-801; Miltenyi Biotec). The cell suspension is then loaded onto a MACS column which is placed under the magnetic field of a MACS Separator. The magnetically labeled CD45+ cells are retained on the column. The unlabeled cells are depleted from CD45+ cells as negative cell fraction. After removal of the column from the magnetic field, the magnetically retained CD45+ cells are eluted as positive cell fraction. This CD45 positive cell fraction (5-10×10⁶ cells) is then administered by intraperitoneal injection into mice bearing the same patient tumor.

### 3. TIL extraction after 3 -4 weeks.

Three to four weeks after CD45 positive cell injection, mice are sacrificed and pancreatic tumor is extracted.

### 4. TIL purification and expansion.

Human TILs from the mouse xenograft tumor are extracted and expanded using well known methods described elsewhere (Meng Q, et al: J. Immunother., 39: 81-9, 2016.).

Initial TIL Culture: Tumor tissue from the patient derived xenograft is minced. Individual single tumor fragments (1-2 mm³) are placed in tissue culture plates along with 1 mL of TIL medium (comprising a cytokine cocktail). Plates are then incubated at 37°C and 5% CO₂ up to 80% expansion per well of TILs.

TIL Expansion Protocol: TILs are further expanded using OKT3 (Biolegend, CA) in the presence of a 1:10 ratio of 55 Gy irradiated allogeneic feeder cells from healthy volunteers. TILs are then expanded in GRex flasks (Wilson Wolf, New Brighton, MN) using TIL medium.

### Example 2: Adoptive cell transfer therapy (ACT) with lymphocytes capable of infiltrating a solid tumor.

Adoptive cell transfer therapy (ACT) with TILs obtained by the method of example 1 is performed as described elsewhere (Besser MJ, et al., Clin Cancer Res 16:2646-55, 2010). Briefly, patients receive a chemotherapy conditioning schedule consisting of cyclophosphamide (60 mg/kg per day for 2 days) followed by fludarabine (25 mg/m2 per day for 5 days). Afterwards, TILs are administrated intravenously followed by high-dose IL-2 (720,000 IU/kg) intravenously three times a day for a maximum of 12 doses.

## Claims

1. A method for the *ex vivo* preparation of lymphocytes capable of infiltrating a solid tumor in a human subject, comprising:
(a) administering CD45+ cells from said subject to an immunodeficient non-human organism, wherein the non-human organism contains a xenograft of said solid tumor generated by implanting cells or tissue from the tumor into the non-human organism;
(b) maintaining the non-human organism for sufficient time for the CD45+ cells to infiltrate the tumor xenograft; and
(c) recovering lymphocytes from the xenograft.

2. The method of claim 1, wherein the solid tumor is selected from the group consisting of lung, genitourinary, gynecologic, breast, head and neck, central nervous system, kidney, pancreatic, and any other gastrointestinal tumor.

3. The method of any of claims 1 or 2, wherein the immunodeficient non-human organism lacks T and B lymphocytes and Natural Killer (NK) cells.

4. The method of any of claims 1 to 3, wherein the immunodeficient non-human organism is a NOD/SCID/IL2λ receptor null (NSG) mouse.

5. The method of any of claims 1 to 4 wherein the xenograft is implanted in the non-human organism in the vicinity of the organ in which the tumor is found in the human subject, in of the organ in which the tumor is found in the human subject or subcutaneously.

6. The method of any of claims 1 to 5, wherein the recovery step (c) is performed at least 3 weeks after the administration step (a).

7. The method of any of claims 1 to 6 further comprising the purification, expansion and/or activation of the lymphocytes obtained in step (c).

8. The method of claim 7 wherein the expansion of the lymphocytes is carried out by stimulating the lymphocytes with a cytokine combination comprising IL-2, IL-15, and IL-21

9. The method of claims 7 or 8 wherein the activation of the lymphocytes is carried using an anti-CD3 antibody in the presence of irradiated feeder cells.

10. A cellular population of lymphocytes obtainable by the method of any of claims 1 to 9.

11. The cellular population of claim 10, wherein the lymphocytes are tumor-specific T cells.

12. The cellular population of any of claims 10 or 11 for use in the treatment of cancer.

13. The cellular population for use according to claim 12 wherein the cancer is pancreatic cancer.

14. The cellular population for use according to claims 12 or 13 wherein the patient is preconditioned by anti-tumor therapy prior to the administration of the cellular population of tumor infiltrating lymphocytes.

15. A method for the *ex vivo* activation of lymphocytes against a tumor found in subject, comprising:
(a) administering CD45+ cells from said subject to an immunodeficient non-human organism, wherein the non-human organism contains a xenograft of said solid tumor generated by implanting cells or tissue from the tumor into the non-human organism and
(b) maintaining the non-human organism for sufficient time for the lymphocytes present in the CD45+ cells to infiltrate the tumor xenograft and to be activated.
